# EUROPEAN PATENT APPLICATION

(11) **EP 1 508 793 A1**
(43) Date of publication of application: **23.02.2005**
(21) Application number: 03728139.1
(22) Date of filing: 27.05.2003
(51) Int. Cl.: G01N 5/02, G01N 33/53, G01N 33/543

(54) **METHOD FOR MEASURING TRACE AMOUNT OF MATERIAL BY USE OF QUARTS RESONATOR**

(30) Priority: 28.05.2002 JP 2002154566
(71) Applicant: National Institute of Advanced Industrial Science and Technology, Tokyo 100-8921 (JP)
(72) Inventor: Kurosawa, Shigeru, Nat. Inst. of, 1-1-1 Higashi, Tsukuba-shi (JP)
(74) Representative: Forstmeyer, Dietmar, Dr. rer. nat., Dipl.-Chem.
(86) International application number: PCT/JP2003/006612
(87) International publication number: WO 2003/100386

(57) **Abstract**

A measurement method which contains:
immobilizing a sample on a quartz oscillator which has a recognition element having a specific binding ability to a particular analyte, and a stabilizer for the said recognition element, which stabilizer comprises a synthetic polymer, immobilized thereon;
measuring the amount of change in the frequency;
comparing the results with the amount of change in the frequency of a quartz oscillator obtained by using a competitive standard substance; and thereby
measuring an amount of said analyte in the measured sample.

## Description

### TECHNICAL FIELD

The present invention relates to a simple and extremely precise method for measuring a concentration of a trace substance in an environment. More particularly, the present invention relates to a measurement that uses a quartz oscillator, and competitive reaction in an antigen-antibody reaction between a protein-binding dioxin complex and dioxins, and that measures a trace dioxin level present in the environment easily, as well as a measurement method for obtaining a toxicity equivalency (TEQ) of dioxin.

### BACKGROUND ART

In recent years, environmental contamination by environmental hormones and others, from dioxins and the like, has become a serious social problem. To preserve the earth environment, high-grade chemical measurement skill is required that can measure chemical substances at the level from ppm to ppt, in order to investigate the actual condition of outbreak circumstances and exposure circumstances of these environmental contaminants. Moreover, to improve monitoring precision, both samples for measurement, and numbers of measurements performed, need to be increased.

Because there are many isomers and homologues of dioxins, and dioxins exist at very low levels in the environment, they are not easy to analyze. Currently, a gas chromatography / mass spectrometry (GC/MS) method is the most reliable method, and it is used widely to officially measure dioxins. In the GC/MS method, a target substance is detected by ionizing and mass analyzing after separating evaporated sample components by gas chromatography.

Despite the reliability of measured values by the GC/MS method, the equipment and exclusive reagents are expensive. Moreover, sample pretreatment requiring skillful technique is necessary. That is, it is necessary to remove measurement disturbing substance as much as possible before injection into an analyzer, and further, it is necessary to concentrate the dioxins in advance because of the extremely low, trace amounts of dioxins in a sample. various special glass equipment is used in these pretreatments, which are performed manually over a period of several days by a skillful analyst with high-grade technique. As a result, the GC/MS method requires much time and cost for analysis, and there has been a limitation in raising the frequency of monitoring.

A simpler, quicker, and less expensive method than the GC/MS method is Enzyme-Linked Immunosorbent Assay (ELISA method), which utilizes antigen-antibody reaction as a focus. The ELISA method is used to detect the presence of an antibody or an antigen utilizing an enzyme activity, using, as a probe, an antigen or an antibody bound to the enzyme via a covalent bond.

Despite that the ELISA method is simpler, quicker, and less expensive than the GC/MS method, the lowest level the ELISA method can measure is of ng/ml units (ppb unit), while the GC/MS method can measure at a level of pg/ml units (ppt unit). As a result, the ELISA method has been used only semi-quantitatively, to monitor at a screening level, for the need for precision analysis. Additionally, labeling treatment of antibody or antigen was necessary in the ELISA method.

On the other hand, as a measurement method for a trace constituent that utilizes biological affinity, such as immune reaction and others, a biosensor that utilizes a surface plasmon resonance (SPR) method (Karube et al., Analytica Chimica Acta, 304 (1995) 139-145; Karube et al., Analytica Chimica Acta, 434 (2001) 223-230), and a measurement method that uses a quartz oscillator, are under research.

A measurement method that uses a quartz oscillator can detect and measure a trace constituent by adsorbing substance to the surface of a quartz oscillator, and using the change in frequency of the quartz oscillator, which is in proportion to the weight of the adsorbed substance. As a result, labeling treatment of an antibody, secondary antibody, or antigen, which are used for measurement with a radioactive isotope, dye, fluorescent substance, enzyme, radical probe, and the like, are entirely unnecessary, making easy, quick, and inexpensive measurement possible.

The quartz oscillator is used as a highly precise and stable oscillating element in many electronic circuits, such as clocks, computers, and communicating/measuring equipment. The quartz oscillator is, moreover, known as the Quartz Crystal Microbalance (QCM), since the quartz oscillator enables highly sensitive analysis, on the order of from the nanogram to the femtogram, by transforming the weight of adhesion on its electrode into a change of oscillatory frequency quantitatively, and it is used as a biosensor, in addition to as a film thickness meter and a chemical sensor.

A usual measurement method for a trace constituent using a quartz oscillator is to measure reactivity between an analyte and a recognition element, and the mass of the analyte, by having a recognition element adsorbed/immobilized on the surface of a quartz oscillator beforehand, and having it react with an analyte, and obtaining the change in frequency before and after the reaction.

As a method for measuring an antigen-antibody reaction that uses, as a recognition element, an antibody that is able to bind specifically to an analyte as an antigen, thereby combining the recognition element with a quartz oscillator, for example, the methods mentioned below are suggested.

In a method disclosed in JP-A-63-11835, a quartz oscillator biosensor, on the surface of which an antigen or antibody was immobilized, is employed, in which method the sensitivity of the quartz oscillator biosensor was amplified by binding, via an antigen-antibody reaction, an antibody- or antigen-immobilizing latex or other microparticle further to a substrate bound via an antigen-antibody reaction.

On the other hand, in a method disclosed in JP-A-03-77601, an antigen or an antibody is reacted in a liquid medium with an antibody- or antigen-supporting insoluble carrier particle toward this antigen or antibody, while allowing a quartz oscillator having no such reaction-participating antigen or antibody immobilized thereon, to be oscillated in the liquid medium, thereby measuring the change in the oscillatory frequency of the quartz oscillator attributable to clot formation caused by the reaction.

However, in any of the methods disclosed in JP-A-63-11835 and JP-A-03-77601, it is essential for the antigen to be a polymer comprising multiple antibody binding sites. Thus, when an analyte is a low-molecular-weight substance, such as an environmental contaminant, including a dioxin and the like, or when an analyte has only one antibody binding site, the latex on which an antigen or antibody is immobilized cannot bind to an analyte, or the insoluble carrier on which antigen or antibody is immobilized forms a clot, to become unable to bind to a quartz oscillator, resulting in difficulty in the measurement.

As an example, to solve these problem, a method which comprises using competitive reaction, allowing multiple analytes to be bound to protein polymers, to form a competitive standard substance that is allowed to compete with an analyte in the test sample, was disclosed in Japanese Patent Application Nos. 2001-117308 and 2001-117309, and also in Xi Chun Zhou et al., Analyst, 2001, 126, pp.71-78. According to the method disclosed in these documents, a larger amount of the analyte present in the test sample leads to more efficient prevention of the binding of the competitive standard substance to the recognition element on the surface of the quartz oscillator, resulting in a reduced change in frequency. Therefore, even when the measurement target is a low-molecular-weight substance, or when it is a substance comprising only one antibody binding site, highly sensitive and highly precise measurement is possible.

However, either measurement method using a quartz oscillator mentioned above measures a model solution that comprises a simple substance. In the real-world environment, multiple substances coexist. For example, in the model solution for dioxins, there exists only 2,3,7,8-tetrachlorodibenzoparadioxin (2,3,7,8-TCDD), but in an actual environmentally-derived sample (for example, an extract from fly ash of an incineration plant), there are more than 100 multi-component dioxins and furans, at varying concentrations, coexisting in the sample.

On the other hand, during the antigen-antibody reaction, phosphate buffered saline (PBS) is used as a solvent, but when substances that are insoluble in water, such as dioxins, are the measurement target, dimethyl sulfoxide (DMSO) and a surfactant (a phosphate buffer with 0.05% of Tween 20 (a trade name, manufactured by Sigma Chemical Co.)) are mixed with PBS, to make the measurement target soluble. In this case, as the higher structure of the antibody that was immobilized on a quartz oscillator changes under the influence of DMSO and a surfactant, it will not be able to recognize the particular analyte alone, and therefore a stabilizer will be used, to maintain the higher structure of the antibody. As this stabilizer, and accordingly, bovine serum albumin (BSA), casein hydrolysate, modified protein, or such were used so far.

However, when measuring an environmental sample, the stabilization effect of conventional stabilizers was low, and the effect could not maintain the higher structure of the antibody; and, accordingly, even if an antibody that could bind specifically to an analyte, which behaves as an antigen, was used, an antigen-antibody reaction with a substance similar to the analyte might occur, and therefore it was difficult to conduct accurate measurement in a mixture system of an environment that includes multiple components. For example, when the 2,3,7,8-TCDD in an environmental sample was treated as a measurement target, a monoclonal antibody, which exhibits selectivity only to 2,3,7,8-TCDD, could not recognize the measurement target normally, and therefore it was unable to bring about accurate measurement. As a result, there are no reports of environmentally-derived samples actually having been measured by a method using a conventional quartz oscillator.

Moreover, when screening (environmentally monitoring) dioxins, it was necessary to make a large amount, corresponding to the number of analytical test samples, of anti-dioxin antibody-immobilizing quartz oscillators, that had to be stored in order to analyze many test samples at once.

However, since it was not possible to store quartz oscillators for a long time when using a conventional stabilizer, because the higher structure of the antibody deteriorated over time, and the particular analyte alone could not be recognized, and therefore, accurate measurement could not be performed.

On the other hand, because dioxins differ in toxicity depending on the dioxin variety, from the viewpoint of environmental monitoring, toxicity equivalency (TEQ) in the environment is important. Here, toxicity equivalency is derived by multiplying an actually measured concentration by a toxicity equivalent coefficient. However, because it has been difficult to measure a mixture system of an environment that includes multiple components accurately by the conventional measurement method using a quartz oscillator, it has also been difficult to measure toxicity equivalency accurately.

Other and further features and advantages of the invention will appear more fully from the following description, taken in connection with the accompanying drawings.

### BRIEF DESCRIPTION OF THE DRAWINGS

Fig. 1 is a front view showing the relation between the recognition element (antibody) placed on a quartz oscillator and the stabilizer.
Fig. 2 is a diagram showing the relation between the concentration of 2,3,7,8-TCDD and the change in oscillatory frequency of a quartz oscillator of the present invention.
Fig. 3 is a diagram showing the correlation between a measured result by the method of the present invention and a measured result by the ELISA method.
Fig. 4 is a diagram showing the correlation between a measured result by the method of the present invention and a measured result by the GC/MS method.

### DISCLOSURE OF THE INVENTION

The present invention resides in a method for measuring a concentration of dioxins in an environmentally-derived sample or a biologically-derived sample, which comprises: using a quartz oscillator; and a method for measuring a toxicity equivalency thereof.

### BEST MODE FOR CARRYING OUT THE INVENTION

The present invention will be explained further in the following.

That is, the present invention provides the following:
(1) A measurement method which comprises:
   immobilizing a sample on a quartz oscillator which has a recognition element having a specific binding ability to a particular analyte, and a stabilizer for the said recognition element, which stabilizer comprises a synthetic polymer, immobilized thereon;
   measuring the amount of change in the frequency;
   comparing the results with the amount of change in the frequency of a quartz oscillator obtained by using a competitive standard substance; and thereby
   measuring an amount of said analyte in the measured sample.
(2) A measurement method for measuring a trace constituent in an environmentally-derived sample or a biologically-derived sample, which comprises:
   immobilizing a sample on a quartz oscillator which has a recognition element having a specific binding ability to a particular multi-component analyte, and a stabilizer for the said recognition element, which stabilizer comprises a synthetic polymer, immobilized thereon;
   measuring the amount of change in the frequency;
   comparing the results with the amount of change in the frequency of a quartz oscillator obtained by using a competitive standard substance; and thereby
   measuring an amount of said analyte in the measured sample.
(3) A measurement method according to item (1) or (2), which comprises: using a clot element having a recognition element immobilized on an insoluble carrier particle; allowing said clot element and the recognition element on the quartz oscillator to bind specifically to an analyte; and thereby amplifying and measuring the change in the oscillatory frequency.
(4) A measurement method according to any one of items (1) to (3), wherein the analyte is dioxins, furans, and brominated dioxins that are environmentally derived or biologically derived.
(5) A measurement method according to any one of items (1) to (4), wherein the analyte is dioxins that are environmentally derived or biologically derived, and the recognition element is a monoclonal or polyclonal antibody that can bind specifically to dioxins regarded as antigens.
(6) A measurement method according to item (5), wherein the recognition element is IgG, IgM, Fab, F(ab')₂, or ScFv, which are monoclonal or polyclonal antibodies.
(7) A measurement method according to any one of items (4) to (6), which comprises:
   immobilizing a monoclonal or polyclonal antibody, which can bind specifically to dioxins regarded as antigens, on a quartz oscillator, in the coexistence of a stabilizer comprising a synthetic polymer;
   after storage for several days, conducting an antigen-antibody reaction; and thereby
   measuring the concentration of dioxins.
(8) A measurement method according to any one of items (1) to (7), which comprises using a small-sized sensor system, which is a unification of a dry cell drive-type or a battery drive-type frequency indicator and an oscillating circuit, or a system formed by combining the said sensor system with a computer for collecting data and controlling, to thereby measure the amount of change in the frequency of the quartz oscillator.
(9) A measurement method according to any one of items (1) to (8), which comprises: binding the quartz oscillator to a micro passage, which has an extraction/pretreatment function of the sample, or embedding the quartz oscillator into a micro passage; and using a micro passage-type chemical reaction system, which accelerates the antigen-antibody reaction time in a micro reaction space.
(10) A measurement method according to any one of items (1) to (9), wherein the dioxin, as an analyte, is 2,3,7,8-tetrachlorodibenzoparadioxin.
(11) A measurement method according to any one of items (1) to (10), which comprises extracting an analyte from an environmentally-derived sample or a biologically-derived sample, by a high-speed solvent extraction method, and conducting a concentration/cleanup pretreatment.
(12) A measurement method for measuring toxicity equivalency of dioxins, which comprises measuring the concentration of 2,3,7,8-tetrachlorodibenzoparadioxin, by the method according to any one of claims 1 to 11, and measuring toxicity equivalency of an entire sample.
(13) A biochip, utilized for the method according to any one of items (1) to (12), which comprises a quartz oscillator which has a recognition element having a specific binding ability to a particular analyte, and a stabilizer for the said recognition element, which stabilizer comprises a synthetic polymer, immobilized thereon.

The present invention will be explained in detail in the followings.

At first, the method for measuring the concentration of the present invention will be explained.

The present invention is characterized by using a stabilizer which comprises a particular synthetic polymer, and immobilizing a recognition element having a specific binding ability to an analyte contained in a contaminated material of multiple components, on a quartz oscillator, and binding multiple analytes to a polymer protein and the like, and regarding this as a competitive standard substance, and using the competitive reaction to compete it with the analyte in the test sample. In the present invention, by using a stabilizer comprising a particular synthetic polymer, it is possible to measure the concentration of a mixture system comprising multiple components accurately which was unable to measure by a conventional method using a quartz oscillator.

The antigen recognition of antibody has a secondary or a tertiary higher structure of a steric structure of a target molecule, and it is particularly necessary to stabilize the tertiary higher structure which is composed by amino acid sequence of hind of the tip of Fab portion where it is an antigen recognition site of the antibody. And, it is also necessary to stabilize the structure of the antibody against all sorts of external changes (temperature, dehydration, pH, ion intensity). The stabilizer which is used in the present invention is able to stabilize these.

In the present specification, the stabilizer (also referred to as "blocking agent") is a reagent which provides effects of (1) decrease in non-specific adsorption of antigen (the selectivity, the improvement of sensitivity) and (2) maintenance of a higher structure which is the foundation of molecule recognition ability of antibody (the stability of the antibody, the improvement of endurance) during an immune sensor or during an antigen-antibody reaction of an enzyme immobilized immunoassay, a solid phase radioimmunoassay or the like.

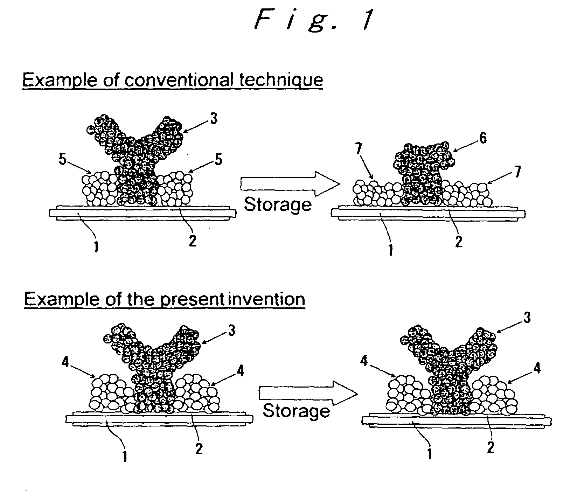

Fig. 1 is an explanatory drawing showing the relation between the recognition element (antibody) placed on a quartz oscillator and the stabilizer. The upper row of the Fig. 1 shows an example of a conventional technique and the lower row shows the example of the present invention. The difference between the conventional stabilizer and the stabilizer of the present invention will be explained below by referring to Fig. 1.

At the surface of gold electrode 2 of quartz oscillator 1 where antibody 3 was fixed on, the stabilizer 5 which was able to use conventionally was physically adsorbing or chemically binding to the space where the antibody 3 was not immobilized on, and was performing the role of excluding (or restraining) the coexisting protein in the sample, besides an analyte, to adsorb non-specifically on the portion of quartz oscillator 1 where antibody 3 was not immobilized on. According to this, it was able to obtain a net response of an antigen-antibody reaction alone on the quartz oscillator. This action is applicable similarly to the stabilizer 4 used in the present invention.

However, because a conventional stabilizer 5 was mostly a biologically-derived product such as BSA and a denatured protein, there was no stabilization for itself, and accordingly the stabilizer 5 denatured into stabilizer 7 as the time passes by, and it could not maintain the higher structure of antibody 3. Due to this, antibody 3 was denatured into antibody 6 and react with substances besides a particular analyte, and accordingly it was not able to measure a particular analyte alone accurately.

Comparing to this, since the stabilizer 4 itself, which is used in the present invention, has endurance against the elevation of the temperature, a change in time, a change in pH and the like, the activity of antibody 3 declines little against the rise in temperature, a change in time, a change in pH and the like, and accordingly the stabilized higher structure of antibody 3 will be maintained, and it is able to be preserved for several days in the state of which antibody 3 immobilized on quartz oscillator 1.

For the stabilizer, a compound which has a chemical structure similar to biomembrane is used from the viewpoint of maintaining the higher structure of antibody. For example, a water soluble polymer composed from units of a phosphoryl choline group-containing compound, which comprises a structure similar to a polarity group of lipid that is composing a cell membrane, and the like are mentioned, and for a specific example, a polymer composed from units of 2-methacryloyl oxyethyl phosphoryl choline (MPC) and the like are mentioned. MPC polymer can be a homopolymer, a random copolymer, a block polymer, a graft polymer, an acrylate derivative of macromonomer, an ethyl fumarate derivative, a styrene derivative and the like. MPC polymer can be manufactured by the method described in JP-A-06-313009, JP-A-10-296063, JP-A-2001-261740 and the like.

These compounds, which comprise a chemical structure similar to a biomembrane, are dissolved in a PBS solution and are used as a stabilizer. For specific examples of the stabilizer, Block Ace (a trade name, manufactured by Snow Brand Milk Products Co.,Ltd.), Lipidure (a trade name, manufactured by NOF Corporation), or StabilGuard (a trade name, manufactured by SurModics, Inc.) is mentioned.

The concentration and the amount of a stabilizer used may vary depending on the type of the stabilizer, and in case of StabilGuard (a trade name), an original solution is used as it is, and in case of Lipidure (a trade name), it is used as a solution at 0.01 to 25% by mass, preferably 0.1 to 5% by mass, more preferably 0.2% by mass in 10mM of PBS (pH 7.0) and also it is preferable to use 0.01 to 0.05mL, more preferably 0.015 to 0.03mL for the electrode of a quartz oscillator (a diameter of the electrode 0.5 cm, area of the electrode 0.196 cm²).

Usually, a phosphate buffered saline (PBS) is used as a solvent in an antigen-antibody reaction, but when a substance which is difficult to solubilize in water, such as dioxins, is a measurement target, it is preferable to mix dimethyl sulfoxide (DMSO) in PBS in order to solubilize the measurement target. DMSO is mixed preferably at 10 to 50%, more preferably at 25% by volume ratio. A surfactant can also be mixed and used in place of DMSO. As the surfactant, for example, a phosphate buffer to which 0.05% of Tween 20 (a trade name, manufactured by Sigma Chemical Co.) is added, can be used and it is mixed preferably at 0.01 to 5%, more preferably at 0.03 to 1% by volume ratio.

An analyte in the present invention will not be restricted particularly, and for example, an environmentally-derived sample such as combustion ash of an incineration plant, fly ash, soil, residue in the interior of a combustor, contaminated soil, condensed deposit, drainage, sewage, ground water, water of rivers/lake/marsh, sludge, and a trace constituent contained in a biologically-derived sample such as cow's milk, mother's milk, blood, urine, body fluid, the cellular tissue, is mentioned. For the trace constituent, for example, environmental contaminants, from the so-called environmental hormones and the like, which were specified as endocrine disruptors such as coplanar PCBs, furans, dioxins, brominated dioxins, and a trace hormones in human body and the like, is mentioned. As used herein, the term "dioxins" is a polychlorinated dibenzo-p-dioxin (PCDD) and polychlorinated dibenzofuran (PCDF), and as a specific example, 2,3,7,8- tetrachlorodibenzoparadioxin and 2,3,7,8- tetrachlorodibenzofuran are mentioned.

In the present invention, it can be measured with high sensitivity even when the analyte is a low molecular weight substance, which has about 100 to 1000 of molecular weight, or a substance which only has one antibody binding site.

In the present invention, multiple analytes are bound to a polymer protein which is used as a competitive standard substance in a reaction allowing it to compete with the analyte in a test sample. The competitive standard substance used in the present invention is multiple analytes bound to a protein polymer.

The protein is not limited particularly, and in view of availability and economy, for example, serum albumin which is a protein having a molecular weight of several ten thousands used widely in the biochemical field as well as an enzyme such as peroxidase, β-galactosidase is used suitably.

As a method for binding multiple analytes to a protein polymer, for example, a mixed acid anhydride method, an N-hydroxysuccinimide method, the same reactivity crosslinking method, an N-(m-maleimidobenzoyloxy) succinimide-type crosslinking-method or the like is mentioned.

The analyte is measured by using a quartz oscillator having a recognition element immobilized thereon.

For the quartz oscillator used in the present invention, one which was used conventionally can be used, and with regard to the variety of it, for example, AT Cut, GT Cut, BT Cut and the like are mentioned, and the material of an electrode may preferably be gold, silver and the like.

An oscillatory frequency of the quartz oscillator is not limited particularly, and it should be chosen according to the use suitably, and 5 to 50 MHz is preferable, and 9 to 30 MHz is more preferable. When the oscillatory frequency is less than 5 MHz, the sensitivity is not enough, and when it is over 50 MHz, the noise will be generated to affect a practical use.

The biochip is made by immobilizing a recognition element and a stabilizer on a quartz oscillator.

The method for immobilizing a recognition element on a quartz oscillator is not limited particularly, and it can be done by a conventional way. For example, a method involving a glutaraldehyde treatment after a cysteamine treatment over the gold electrode surface of the quartz oscillator followed by allowing the recognition element to be adsorbed on the surface is mentioned.

The recognition element immobilized on the quartz oscillator has a specific binding ability toward an analyte. The recognition element used in the present invention, is not limited particularly as long as the product binds specifically toward the analyte, and for example, an antibody which produces an antigen-antibody reaction with the analyte, a receptor molecule which recognizes the analyte specifically and the like are mentioned.

For the antibody which undergoes an antigen-antibody reaction with an analyte, for example, a monoclonal antibody, a polyclonal antibody (IgG, IgM or the like) or the like made by immunizing animals such as a rabbit, a goat, a sheep, using the an analyte and a haptenized analyte as an antigen, is mentioned. For the antibody, for example, F(ab')₂, Fab', Fab obtained by enzyme treatment and the like can be used, and further, Fab', scFv and the like prepared by a genetic recombination technique can be used.

For the receptor molecule which recognizes an analyte is not limited particularly, and for example, it is known that dioxins bind to an Ah (allyl hydrocarbon) receptor, and accordingly such receptor can be used.

Furthermore, as a recognition element, peptide, DNA or the like which has an ability of binding to an analyte can be used, and a polymer which have a complementary binding site to an analyte that is made by a molecular imprinting method can be used.

The recognition element is immobilized on a quartz oscillator, and it is preferable to form a clot element by immobilizing a free recognition element on insoluble carrier particles. The weight gains by sandwiching the complex of a competitive standard substance between the recognition element immobilized on the quartz oscillator and the clot element, and as a result, the amount of change in the frequency will be amplified and it will be possible to detect/quantitate the analyte at a far lower concentration. When the recognition element is an antibody which undergoes an antigen-antibody reaction with an analyte, the clot element will work as a secondary antibody.

For the insoluble carrier particles used in the present invention, it is not limited particularly as long as it is insoluble substantially in liquid medium used in the present invention, for example, the particles comprised from organic polymer substances such as polystyrene, a styrene-butadiene copolymer, a styrene-sodium styrene sulfonate copolymer, a styrene-methacrylate copolymer, a styrene-chloromethylstyrene copolymer, a chlorinated polystyrene; inorganic oxide microparticles such as silica, silica-alumina; magnetic metallic microparticles such as ferrite coated with an organic thin layer, or the like, is mentioned.

The diameter of the particles can be large as long as it can disperse stably in a liquid medium in order to produce enough amount of change in the oscillatory frequency, and are preferable to be 0.01 µm to 1 µm, more preferable to be 0.05 µm to 0.5 µm.

The method for forming a clot element by allowing a recognition element to be supported by insoluble carrier particles is not limited particularly, and it can be done by a conventional way. For example, a method for chemical adsorption which uses a coupling agent and the like for insoluble carrier particles, a method for chemical adsorption which uses a latex, which is a polymer substance with reactive functional groups on the surface of particles, as insoluble carrier particles, or a method for physical adsorption which utilizes a hydrophobic interaction or the like, is mentioned.

The concentration of the clot element is not limited particularly and can be established suitably, and are preferable to be 0.01 to 1.0% by mass, more preferable to be 0.05 to 0.5% by mass. If it is less than 0.01% by mass, the antigen-antibody reaction will not take place sufficiently, and if it is 1.0% by mass, the clot element will be difficult to disperse stably in a liquid medium.

As an equipment used in the present invention, for example, the equipment described in JP-A-03-77601 can be used. In the measuring equipment, a quartz oscillator tip is connected to a measurement system comprising an oscillating circuit and a microcomputer for counting the frequency and processing the data.

Furthermore, the present invention enables a measurement by using a small-sized sensor system which is a unit integrating a dry cell drive type or a battery drive-type frequency indicator and an oscillating circuit. With this, the measuring equipment can be hand-carried, and the measuring can be done at any place.

Furthermore, the present invention can bring about the measurement by embedding the quartz oscillator into a micro passage, and using a micro passage type chemical reaction system which accelerates the antigen-antibody reaction time of in a micro reaction space. By using this system, the competitive reaction with an analyte in a test portion can be finished within one minute, thereby enabling a much quicker measurement. Specifically, the system described in Kitamori et al., Analytical Chemistry, Vol.73, No. 6, 1213-1218 (2001), Kitamori et al., Analytical Chemistry, Vol.74, No. 9, 2014-2020 (2002) or the like, can be used.

Using a case where a recognition element is an antibody for an example, one embodiment of the measurement method of the present invention will be explained in the followings.

Upon measuring, the environmentally-derived sample or the biologically-derived sample is extracted within a few hours by a high speed solvent extraction method, and a concentration/clean up pretreatment is conducted. The high speed solvent extraction method is described in Kazuto Sakata et al., "A quick dioxin analysis technique which combined high speed solvent extraction method and immunoassay", Journal of Resources and Environment, Vol. 37, No. 9, 87 to 91 (2001). By putting the present invention, combining with this pretreatment, into practice, the result of the analysis can be obtained within four to five hours from collecting samples while it took two weeks from collecting samples to measurement by a conventional official method.

First, the quartz oscillator, which was conducted a variety of chemical treatments to immobilize an antibody and the like, was immersed in a liquid medium of a buffer solution or the like, while allowing the liquid medium to be stirred preliminarily at a constant speed by a stirrer which was put in a thermostat cell for the measurement. Alternatively, the quartz oscillator, which has been subjected to a variety of chemical treatments to immobilize an antibody and the like, was put in a thermostat cell for measurement and the antibody immobilization operation was conducted as mentioned below.

For examples of the liquid medium here employed in the present invention, a phosphate buffer solution, a glycine buffer solution, a tris hydrochloric acid buffer solution and the like are preferable. It is preferable for the rotation speed of the stirrer to be, from the viewpoint of carrying out the antigen-antibody reaction promptly and at the same time the oscillation stably, 500 to 1500 rpm, more preferable is to be 800 to 1000 rpm. It is also preferable to maintain a constant rotation speed during the reaction.

Next, a biochip is made. The quartz oscillator is immersed in the liquid medium in the above-mentioned thermostat cell, and oscillated, and the antibody is supported by the quartz oscillator. Then 0.1 to 5% of the stabilizer is added on the quartz oscillator, and thereafter the stabilizer which is not immobilized on the quartz oscillator is washed off with water. After drying the quartz oscillator, or in the solution, the frequency F1 is measured when the oscillatory frequency is stabilized. In the present invention, after immobilizing antibody on the quartz oscillator by using a stabilizer, followed by a storage for a few days, an antigen-antibody reaction is performed and the concentration of dioxins can be measured. As a result, it can prepare and preserve a large amount of the antibody immobilizing quartz oscillator (biochip), and analyze a large number of samples at once. It is preferable to use the biochip thus produced before the expiration of the storage period. The storage period at 25°C in a dry condition, is preferably 1 to 60 days, more preferably 1 to 7 days. Under a dry condition at 0 to 4°C (refrigerated condition), it is preferably 1 to 1000 days, more preferably 1 to 365 days, and particularly not longer than 30 days.

Next, a mixture of a protein to which multiple antigens bind, and a sample comprising antigen, is added, thereby performing a competitive immune reaction. If an antigen is contained in the test sample at this moment, the binding of the protein having multiple antigens bound thereto to an antibody supported on a surface of the quartz oscillator will be inhibited. Because the frequency of the quartz oscillator will decrease depending on the weight of the substance bound on the quartz oscillator, a decrease in the weight of the antigen bound protein adsorbing to the quartz oscillator leads to decrease in the frequency reduction.

The free antigen-binding protein, which does not bind to the antibody on the surface of the quartz oscillator, is removed and washed off. While stirring continuously, the frequency which is once stabilized constantly is measured as a value F2. The amount of change in the frequency, ΔF, is calculated by subtracting F2 from the obtained F1.

Although the temperature of the reaction during the above-mentioned measurement can be room temperature, it is preferable, in order to establish a high cycle stability of the quartz oscillator, to operate the temperature under the circumstance of 25 to 37°C with precision of ±0.1°C.

In present invention, the calibration curve is made by using an analyte of a known concentration, and by using this, the concentration of an analyte in the test sample is calculated.

While the quartz oscillator used in the present invention can be used again by washing and removing an antibody and the like immobilized on the surface with a mixed solution of sulfuric acid/aqueous hydrogen peroxide, a potassium permanganate solution or the like, this quartz oscillator can be exchanged after each measurement since it is as inexpensive as about 100 yen.

Particularly in the case of analyzing dioxins, because the toxicity of dioxins is very high, and the solution after washing must be combusted and decomposed at a high temperature of 1500°C or higher after concentration, it is preferable to basically use a quartz oscillator as a disposable product.

The measurement method of a toxicity equivalency of the present invention will be explained below.

In case of dioxins, by multiplying a concentration of each component by a designated toxicity equivalent coefficient, the toxicity equivalency (TEQ) of each component can be calculated. The toxicity equivalent coefficient is prescribed by World Health Organization/International Programme on Chemical Safety (WHO/IPCS).

The toxicity equivalent coefficient of 2,3,7,8-tetrachlorodibenzoparadioxin (2,3,7,8-TCDD) is 1, and it is known that a high toxicity equivalent coefficient is associated when compared to other dioxins in the environment. Also, it is known that the concentration of 2,3,7,8 -TCDD in the environment is higher than those of other dioxins. Therefore, by measuring the concentration of 2,3,7,8 -TCDD according to the above-mentioned method of the present invention, and from the calibration curve which is made beforehand by using a GC/MS method, it will be possible to measure the toxicity equivalency of entire dioxins in the sample. This toxicity equivalency measurement method can be applied to an environmentally-derived sample and a biologically-derived sample.

### Examples

The present invention will be described in more detail based on the following examples. However, the present invention is not limited by these examples.

### Example 1

### <Anti 2,3,7,8-TCDD-antibody immobilization and stabilization treatment of a quartz oscillator >

A test was carried out by regarding 2,3,7,8-TCDD, which is a low molecular weight compound, as an analyte.

A quartz oscillator of AT Cut (9 MHz) was used. At first, the gold electrode surface of the quartz oscillator was washed with a mixed solution of sulfuric acid/aqueous hydrogen peroxide. After washing with purified water, nitrogen was circulated on the quartz oscillator at 2.5 L/minute for drying. Next, the gold electrode surface of the quartz oscillator was treated with 0.01M of cysteamine, and was treated with a 1% solution of glutaraldehyde continuously, and was washed three times with 1 mL of purified water after activation for antibody-binding.

Next, by adding 30 µL of a PBS solution (pH 7.4) of 50 µg/mL of an anti 2,3,7,8-TCDD monoclonal antibody on the quartz oscillator, and by reacting for 90 minutes at 25°C, the antibody is immobilized on the quartz oscillator. After washing three times with 1 mL of purified water, 30 µL of a 0.02M glycine solution was added on quartz oscillator in order to cap the remaining unreacted aldehyde group on the quartz oscillator, and a reaction was conducted for 30 minutes at 25°C. After washing three times with 1 mL of purified water, 30 µL of a 0.2% stabilizer-PBS solution was added on the quartz oscillator, and a reaction was conducted for 90 minutes at 25°C. Lipidure-HM (a trade name, manufactured by NOF Corporation) was used for a stabilizer. After washing three times with 1 mL of purified water, nitrogen was circulated on the quartz oscillator at a flow rate of 2.5 L/minute for drying, and 60 minutes later, the oscillatory frequency of the antibody-immobilizing quartz-oscillator was measured, and the measured value was designated as F1.

### <Antigen-antibody reaction I>

A DMSO solution of 2,3,7,8-TCDD was mixed into 10 mM of PBS solution (pH 7.4) of 100 ng/mL of 2,3,7,8-TCDD-binding ovalbumin (manufactured by Research Diagnostics, Inc.) in the ratio of 1:3, and it was regarded as 25% of DMSO solution. Antigen solutions of competitive reaction were prepared by a dissolution at the concentrations of 0.001, 0.01, 0.1, 1, 10 and 100 ng/mL of 2,3,7,8-TCDD.

10 µL of each antigen solution was added to an anti-2,3,7,8-TCDD antibody-immobilizing quartz oscillator which was prepared as mentioned above, and was reacted for 60 minutes at 25°C, the antigen-antibody reaction I was performed. Since the antigen-antibody reaction is as substantial as about 300 Hz, the reaction can be finished in 10 minutes of the reaction time if it is judged that the antigen-antibody reaction rate of 65% was sufficient. After completion of the reaction followed by washing three times with 1 mL of purified water, nitrogen was circulated on the quartz oscillator at a flow rate of 2.5 L/minute for drying, and after 30 minutes the oscillatory frequency of the antibody-immobilizing quartz-oscillator was measured and the measured value was designated as F2.

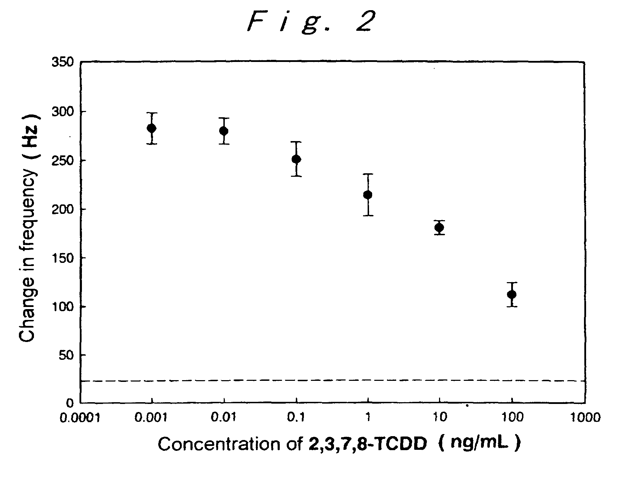

The value calculated by subtracting F2 from F1 is designated as ΔF1, and the results are shown in Table 1. Further, the relationship with the concentration of 2,3,7,8-TCDD is shown in Fig. 2, and used as a calibration curve for measuring the concentration of the dioxins contained in a sample extracted from fly ash of an incineration plant of Example 1 mentioned below.

**Table 1**

| 2, 3, 7, 8-TCDD (ng/mL) | Δ F 1 (Hz) |
|---|---|
| 0 | 280±46 |
| 0. 001 | 282±16 |
| 0.01 | 279±13 |
| 0. 1 | 250±18 |
| 1 | 214±21 |
| 10 | 180±7 |
| 100 | 112±13 |

As is apparent from Table 1, a trace concentration of 2,3,7,8-TCDD as low as 0.001 ng/mL (1 pg/mL) to 100 ng/mL could be measured in the present invention.

### Example 2

### <Antigen-antibody reaction II (sensitization reaction by latex reagents)>

A solution was prepared to be 1 ng/mL by dissolving 2,3,7,8-TCDD (manufactured by Research Diagnostics, Inc.) into 1 ng/mL of 2,3,7,8-TCDD-binding ovalbumin (manufactured by Research Diagnostics, Inc.) solution, and this was regarded as an antigen solution. Antigen-antibody reaction I was peformed in the same manner as in Example 1, and then antigen-antibody reaction II was conducted by adding 10 µL of anti 2,3,7,8-TCDD monoclonal antibody immobilized anti 2,3,7,8-TCDD latex suspension (solid portion 0.01%) into latex for immune reagents (manufactured by Sekisui Chemical Co., Ltd.) after washing, and incubating for 60 minutes at 25°C. After completion of the reaction followed by washing with purified water, the oscillatory frequency of the antibody-immobilizing quartz-oscillator was measured, and the measured value was designated as F3.

The value calculated by subtracting F3 from F2 is regarded as ΔF2. The results are shown in Table 2.

For reference, a 1 ng/mL solution alone of 2,3,7,8-TCDD-binding ovalbumin (manufactured by Research Diagnostics, Inc.), which is a competitive standard substance, was handled as an antigen solution and was tested in the same manner. The results are shown in Table 2.

### Comparative example 1

The test was conducted in the same manner as in Example 2, except that the latex reagents and the stabilizer were not used. The results are shown in Table 2.

**Table 2**

| | | Δ F 2 (Hz) | |
|---|---|---|---|
| | | TCDD-ovalbumin (1ng/ml) | TCDD-ovalbumin (1ng/ml) + TCDD (1ng/ml) |
| Example 2 | With the latex | 932 | 510 |
| Comparative example 1 | Without the latex | 86 | 42 |

As is apparent from Table 2, the measured value of change in the frequency became higher by ten times or more by adding the latex particles, and accordingly a measurement at a high sensitivity is possible.

### Example 3

Antigen-antibody reaction measurement for 2,3,7,8-TCDD, contained in a sample extracted from fly ash of an incineration plant, was tested. Similarly to Example 1, the anti-2,3,7,8-TCDD antibody was immobilized on the quartz oscillator and treated for stabilization, and a competitive reaction solution was prepared at 1 ng/mL by dissolving the sample extracted from fly ash of the incineration plant in a 100 ng/mL solution of 2,3,7,8-TCDD-binding ovalbumin (manufactured by Research Diagnostics, Inc.) as a standard of competitive reaction, and this was regarded as an antigen solution. By using this, the antigen-antibody reaction was conducted in the same manner as in Example 1. The results are shown in Table 3.

### Comparative example 2

The test was conducted in the same manner as in Example 3, except that a stabilizer was not used. The results are shown in Table 3.

**Table 3**

| | Stabilizer | Change in the oscillatory frequency [Hz] |
|---|---|---|
| Example 3 | 0.2% of Lipidure-HM-PBS solution (30 µL) | 200 ± 29 |
| Comparative example 2 | None | 20 ± 45 |

As is apparent from Table 3, when measuring environmental sample which is a mixture, and in the case that the stabilizer was not added, the response of change in the oscillatory frequency decreases remarkably. Compared to this, in the Example of the present invention, which the stabilizer was added, the measured value of change in the frequency became higher by ten times or more when comparing with the case where the stabilizer was not added, and accordingly it was able to measure with high sensitivity.

In the case where the stabilizer is not added, an inhibition by a highly concentrated multi-component substances contained in an actual environmental sample or a cross reactivity of an antibody itself leads to a reduction in change in the frequency based on the antigen-antibody reaction, which leads to difficulty in obtaining the response stably. Further, in the case that the stabilizer is not added, the mixture of various highly concentrated multi-component substances in the measuring solution such as dioxins, furans, does not allow the anti-2,3,7,8-TCDD monoclonal antibody to maintain the higher structure for undergoing the antigen-antibody reaction normally, possibly resulting in an abnormal frequency response.

### Example 4

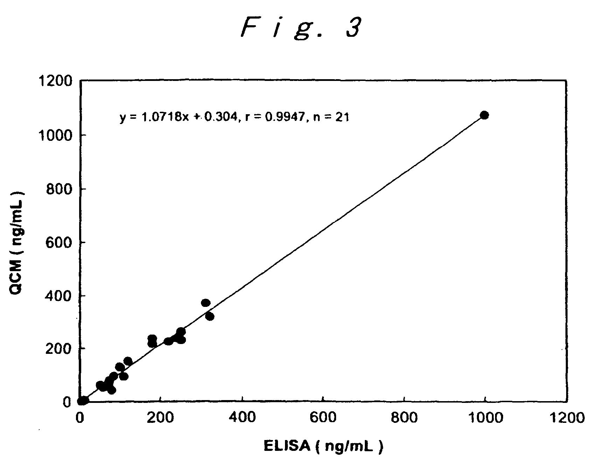

The concentration of 2,3,7,8-TCDD in the sample used in Example 3 was measured by ELISA method, and the correlation with the measured result by the method of the present invention was investigated. The results are shown in Fig. 3. Fig. 3 is a diagram showing a correlation between a measured result by the method of the present invention and a measured result by the ELISA method.

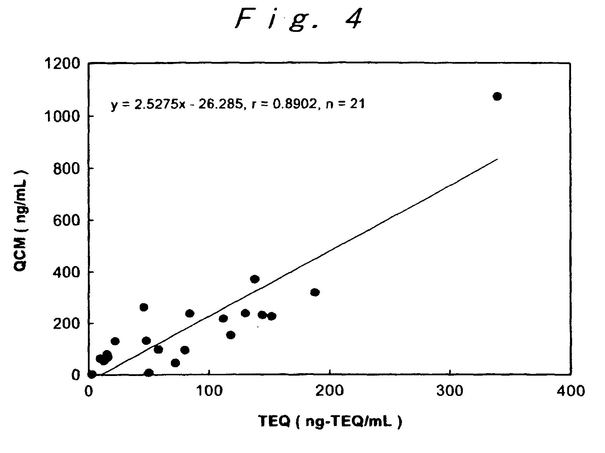

Further, the same sample was measured by a GC/MS method, and the correlation with the measured result by the method of the present invention was investigated. For the measuring equipment, HP-6890/JMS-700 MStation (a trade name, manufactured by JEOL Ltd.), as a gas chromatography/mass spectrometer, and MS-MP8220D (a trade name, manufactured by JEOL Ltd.), as a data processing system, were used. In a GC/MS method, the concentration of individual component of dioxins (such as tetrachlorodibenzoparadioxins (TeCDDs), pentachlorodibenzoparadioxins (PeCDDs), hexachlorodibenzoparadioxins (HeCDDs), heptachlorodibenzoparadioxins (HpCDDs), octachlorodibenzoparadioxins (OCDDs), tetrachlorodibenzofurans (TeCDFs), pentachlorodibenzofurans (PeCDFs), hexachlorodibenzofurans (HeCDFs), heptachlorodibenzofurans (HpCDFs), octachlorodibenzofurans (OCDFs)), which exists in the sample, was measured, and individual toxicity equivalency was calculated by multiplying the concentration of individual component by the individual toxicity equivalent coefficient, and the individual toxicity equivalency was summed up, and the toxicity equivalency (TEQ) of entire dioxins in the sample was calculated. The result is shown in Fig. 4. Fig. 4 is a diagram showing a correlation between a measured result by the method of the present invention and a measured result by the GC/MS method.

As is apparent from Fig. 3, the measured result by the method of the present invention correlates well with the measured result by the ELISA method. Accordingly, the method of the present invention can be utilized as a concentration measurement method in the place of ELISA method. Also as is evident from Fig. 4, the measured result by the method of the present invention also correlate well with the measured result of toxicity equivalency by the GC/MS method. Accordingly, the method of the present invention can be utilized as a toxicity equivalency measurement method in the place of the GC/MS method.

### Example 5

A 100 ng/mL solution of a 2,3,7,8-TCDD-binding ovalbumin (manufactured by Research Diagnostics, Inc.) was regarded as an antigen solution, and in the same manner as in Example 1, anti 2,3,7,8-TCDD antibody was immobilized on the quartz oscillator and treated for stabilization. Subsequently, after storing the sample overnight at 25°C, the measurement experiment of the antigen-antibody reaction of 2,3,7,8-TCDD was conducted in the same manner as in Example 1. The measured result is shown in Table 4.

### Comparative example 3

The sample was prepared and the measurement experiment of the antigen-antibody reaction of 2,3,7,8-TCDD was conducted in the same manner as in Example 5, except that the stabilization and the overnight storage was not conducted. The measured result is shown in Table 4.

### Comparative examples 4 to 6

The sample was prepared and the measurement experiment of the antigen-antibody reaction of 2,3,7,8-TCDD was conducted in the same manner as in Example 5, except that the stabilization was not conducted. It was tested similarly for three times, and each were regarded as comparative examples 4 to 6. The each of the measured results is shown in Table 4.

**Table 4**

| | Details of the experiment | Change in the oscillatory frequency [Hz] |
|---|---|---|
| Example 5 | With the stabilizer, One day storage | 280 ± 15 |
| Comparative example 3 | Without the stabilizer, Without the storage | 304 ± 9 |
| Comparative example 4 | Without the stabilizer, One day storage | 35 ± 34 |
| Comparative example 5 | Without the stabilizer, One day storage | 120 ± 29 |
| Comparative example 6 | Without the stabilizer, One day storage | 84 ± 45 |

As is apparent from Table 4, when an antibody-immobilizing quartz-oscillator for dioxin analysis is preserved overnight, the amount of antigen-antibody reaction decreases remarkably. In this case, when it is without a stabilizer, the change in frequency based on the antigen-antibody reaction is small, and the response is not obtained stably.

On the contrary, an example of the present invention containing the stabilizer exhibited a stable response although it underwent about 10% reduction in the antigen-antibody reaction level after an overnight storage when compared with Comparative example 3 undergoing no overnight storage.

### Example 6

The concentration of 2,3,7,8-TCDD, contained in the sample extracted from fly ash of an incineration plant, was measured in the same manner as in Example 3. The measured result was 97 ng/mL. As a result of applying the measured concentration to the calibration curve shown in Fig. 4, the toxicity equivalency was 49 ng-TEQ/mL.

Using the same sample, the concentration of each component was measured by the GC/MS method. The measured result is shown in Table 5.

**Table 5**

| Target component | Actually measured concentration (ng/ml) | Toxicity equivalent coefficient | Toxicity equivalency(TEQ) (ng-TEQ/ml) |
|---|---|---|---|
| 2,3,7.8-TeCDD | 50 | × 1 | 50 |
| Total of TeCDDs | 54 | --- | --- |
| 1,2,3,7,8-PeCDD | 2.2 | × 1 | 2.2 |
| Total of PeCDDs | 10.2 | --- | --- |
| 1,2,3,4,7,8-HxCDD | 0.46 | × 0.1 | 0.046 |
| 1,2,3,6,7,8-HxCDD | 0.66 | × 0.1 | 0.066 |
| 1,2,3,7,8,9-HxCDD | 20 | × 0.1 | 2.0 |
| Total of HxCDDs | 26 | --- | --- |
| 1,2,3,4,6,7,8-HpCDD | 8.6 | × 0.01 | 0.086 |
| Total of HpCDDs | 12.2 | --- | --- |
| OCDD | 30 | × 0.0001 | 0.0030 |
| Total of entire PCDDs | 132.4 | --- | 54.401 |
| 2,3,7,8-TeCDF | 19.2 | × 0.1 | 1.92 |
| Total of TeCDFs | 24 | --- | --- |
| 1,2,3,7,8-PeCDF | 3.4 | × 0.05 | 0.17 |
| 2,3,4,7,8-PeCDF | 0.54 | × 0.5 | 0.27 |
| Total of PeCDFs | 9.6 | --- | --- |
| 1,2,3,4,7,8-HxCDF | 0.54 | × 0.1 | 0.054 |
| 1,2,3,6,7,8-HxCDF | 0.38 | × 0.1 | 0.038 |
| 1,2,3,7,8,9-HxCDF | 13.4 | × 0.1 | 1.34 |
| 2,3,4,6,7,8-HxCDF | 1.32 | × 0.1 | 0.132 |
| Total of HxCDFs | 14.8 | --- | --- |
| 1,2,3,4,6,7,8-HpCDF | 1.16 | × 0.01 | 0.0116 |
| 1,2,3,4,7,8,9-HpCDF | 7.4 | × 0.01 | 0.074 |
| Total of HpCDFs | 9.4 | --- | --- |
| OCDD | 8.6 | × 0.0001 | 0.00086 |
| Total of entire PCDFs | 66.4 | --- | 4.01046 |
| Grand total of PCDDs and PCDFs | 198 | --- | 58 |

As is apparent from Table 5, the toxicity equivalency of entire dioxins in the sample was 58 ng-TEQ/mL.

Accordingly, by measuring the concentration of 2,3,7,8-TCDD, which is known to present at the most highest concentration and to exhibit the most highest toxicity among the dioxins in the environment, and from the calibration curve which is made beforehand by using the GC/MS method, it was able to measure the toxicity equivalency of entire dioxins in the sample.

According to the present invention, a trace constituent in a contaminated material comprising multiple components, such as an environmentally-derived sample, a biologically-derived sample, can be measured quickly at a high sensitivity by a very easy operation. For example, a trace constituent can be detected by using, as an assay sample, biologically-derived samples such as human serum, urine, body fluid or environmentally-derived samples such as water of rivers/lake, sludge, dust of combustion furnace. Particularly, environmental contaminants, from the so-called environmental hormones and the like, which were specified as endocrine disruptors, such as dioxins, and a trace hormones in human body and the like can be measured with high sensitivity.

A conventional official method of the dioxins measurement (GC/MS method) costed about 250,000 yen per analysis, and its analyzing time took near four weeks. Compared to this, according to the present invention, a measurement is possible at the same precision level as the official method in about four hours at a cost of about 20,000 yen per analysis. Further, with regard to the measuring equipment, the equipment unit of the official method costs 60,000,000 yen whereas it costs about 10,000 yen in the present invention. Accordingly, the present invention is extremely economically favorable.

According to the present invention, by measuring the concentration of highly concentrated and deadly poisonous 2,3,7,8-tetrachlorodibenzoparadioxin (2,3,7,8-TCDD) in the environment, and by calculating toxicity equivalency, it can measure the overall toxicity equivalency of dioxins in a contaminated material comprising multiple components, such as an environmentally-derived sample, a biologically-derived sample.

### INDUSTRIAL APPLICABILITY

A measurement method of the present invention enables a measurement quickly at a high sensitivity by a very easy operation, and accordingly, it is suitable as a measurement method for measuring environmental contaminants including so-called environmental hormones and the like which were prescribed as endocrine disruptors such as dioxins in a contaminated material comprising multiple components such as an environmentally-derived sample and a biologically-derived sample, and a trace hormones in human body, as well as a measurement method for measuring toxicity equivalency thereof.

Having described our invention as related to the present embodiments, it is our intention that the invention not be limited by any of the details of the description, unless otherwise specified, but rather be construed broadly within its spirit and scope as set out in the accompanying claims.

## Claims

1. A measurement method which comprises:
immobilizing a sample on a quartz oscillator which has a recognition element having a specific binding ability to a particular analyte, and a stabilizer for the said recognition element, which stabilizer comprises a synthetic polymer, immobilized thereon;
measuring the amount of change in the frequency;
comparing the results with the amount of change in the frequency of a quartz oscillator obtained by using a competitive standard substance; and thereby
measuring an amount of said analyte in the measured sample.

2. A measurement method for measuring a trace constituent in an environmentally-derived sample or a biologically-derived sample, which comprises:
immobilizing a sample on a quartz oscillator which has a recognition element having a specific binding ability to a particular multi-component analyte, and a stabilizer for the said recognition element, which stabilizer comprises a synthetic polymer, immobilized thereon;
measuring the amount of change in the frequency;
comparing the results with the amount of change in the frequency of a quartz oscillator obtained by using a competitive standard substance; and thereby
measuring an amount of said analyte in the measured sample.

3. A measurement method according to claim 1 or 2, which comprises: using a clot element having a recognition element immobilized on an insoluble carrier particle; allowing said clot element and the recognition element on the quartz oscillator to bind specifically to an analyte; and thereby amplifying and measuring the change in the oscillatory frequency.

4. A measurement method according to any one of claims 1 to 3, wherein the analyte is dioxins, furans, and brominated dioxins that are environmentally derived or biologically derived.

5. A measurement method according to any one of claims 1 to 4, wherein the analyte is dioxins that are environmentally derived or biologically derived, and the recognition element is a monoclonal or polyclonal antibody that can bind specifically to dioxins regarded as antigens.

6. A measurement method according to claim 5, wherein the recognition element is IgG, IgM, Fab, F(ab')₂, or ScFv, which are monoclonal or polyclonal antibodies.

7. A measurement method according to any one of claims 4 to 6, which comprises:
immobilizing a monoclonal or polyclonal antibody, which can bind specifically to dioxins regarded as antigens, on a quartz oscillator, in the coexistence of a stabilizer comprising a synthetic polymer;
after storage for several days, conducting an antigen-antibody reaction; and thereby
measuring the concentration of dioxins.

8. A measurement method according to any one of claims 1 to 7, which comprises using a small-sized sensor system, which is a unification of a dry cell drive-type or a battery drive-type frequency indicator and an oscillating circuit, or a system formed by combining the said sensor system with a computer for collecting data and controlling, to thereby measure the amount of change in the frequency of the quartz oscillator.

9. A measurement method according to any one of claims 1 to 8, which comprises: binding the quartz oscillator to a micro passage, which has an extraction/pretreatment function of the sample, or embedding the quartz oscillator into a micro passage; and using a micro passage-type chemical reaction system, which accelerates the antigen-antibody reaction time in a micro reaction space.

10. A measurement method according to any one of claims 1 to 9, wherein the dioxin, as an analyte, is 2,3,7,8-tetrachlorodibenzoparadioxin.

11. A measurement method according to any one of claims 1 to 10, which comprises extracting an analyte from an environmentally-derived sample or a biologically-derived sample, by a high-speed solvent extraction method, and conducting a concentration/cleanup pretreatment.

12. A measurement method for measuring toxicity equivalency of dioxins, which comprises measuring the concentration of 2,3,7,8-tetrachlorodibenzoparadioxin, by the method according to any one of claims 1 to 11, and measuring toxicity equivalency of an entire sample.

13. A biochip, utilized for the method according to any one of claims 1 to 12, which comprises a quartz oscillator which has a recognition element having a specific binding ability to a particular analyte, and a stabilizer for the said recognition element, which stabilizer comprises a synthetic polymer, immobilized thereon.
